# EUROPÄISCHE PATENTSCHRIFT

(11) **EP 0 699 419 B1**
(45) Veröffentlichungstag und Bekanntmachung des Hinweises auf die Patenterteilung: **17.10.2001**
(21) Anmeldenummer: 95112932.9
(22) Anmeldetag: 17.08.1995
(51) Int. Cl.: A61B 17/64

(54) **Osteosynthetischer Fixateur**
Osteosynthetic fixateur
Fixateur ostéosynthétique

(30) Priorität: 03.09.1994 DE 4431525; 05.08.1995 DE 19528839
(43) Veröffentlichungstag der Anmeldung: 06.03.1996
(73) Patentinhaber: Veith, Wolfgang Dr., D-69124 Heidelberg (DE)
(72) Erfinder: Veith, Wolfgang, Dr., D-69124 Heidelberg (DE)
(74) Vertreter: Sartorius, Peter, Dipl.-Ing.

(56) Entgegenhaltungen:
- EP-A- 0 227 594
- EP-A- 0 424 292
- DE-A- 2 718 515
- FR-A- 2 457 676
- FR-A- 2 665 353

## Beschreibung

Die Erfindung bezieht sich auf einen Fixateur mit einem eine Anlagefläche aufweisenden Tragkörper, auf dem zwei Feststellvorrichtungen zur Aufnahme von jeweils mindestens einem Knochenschrauben-Aufnahmeteil angeordnet sind, wobei jede Feststellvorrichtung eine Drehsicherung und ein gegen axiales Verstellen sicherndes Feststellelement aufweist.

Es sind allgemein Fixateure bekannt, die bei Frakturen, Mehrfragmentfrakturen bzw. bei Distraktionen und Kompressionen einsetzbar sind und hierzu aus einem auf einer Gewindestange angeordneten Zentralelement bestehen, an dem mittels Knochenschrauben-Klemmbacken mindestens zwei Knochenschrauben befestigt sind. Neben dem Tragkörper sind auf dem ein Gewinde aufweisenden Gestänge zwei weitere Knochenschrauben-Klemmbacken angeordnet, wobei zumindest eine Klemmbacke mit einem Kugelgelenk versehen ist, so daß mindestens zwei Knochenschrauben gegenüber den übrigen Knochenschrauben verstellbar sind. Das Verstellen der einzelnen Klemmbacken kann durch eine Stellmutter erfolgen, die hierzu auf dem Gestänge in die entsprechende Richtung gedreht wird, um auf diese Weise zwei mit Abstand zueinander angeordnete Knochenschrauben-Klemmbacken aufeinander zu- oder voneinander wegzubewegen. Ein derartiges Verstellen der Knochenschrauben-Klemmbacken ist sehr zeitaufwendig. Außerdem ist die Anpassung der einzelnen Knochenschrauben bei Mehrfragmentfrakturen an die einzelnen Knochenteile nicht ohne weiteres möglich.

Demgemäß besteht die Erfindungsaufgabe darin, einen Fixateur der eingangs geschilderten Art derart auszubilden, daß eine schnelle Anpassung des Fixateurs bzw. der zugehörigen, die Knochenschrauben aufnehmenden Klemmbacken an das repositionierende Knochenteil mit wenigen Handgriffen möglich ist.

Gelöst wird die Aufgabe erfindungsgemäß dadurch, daß die beiden Feststellvorrichtungen auf der gesamten Länge des Tragkörpers mittels der Anlagefläche drehgesichert sind. Hierdurch läßt sich der Fixateur sehr leicht handhaben und die Knochenschrauben in jede gewünschte Lage verstellen, ohne daß mehrere Handgriffe gleichzeitig notwendig werden. Da beide Feststellvorrichtungen über mindestens eine Drehsicherung bzw. Anlagefläche auf dem Tragkörper über die gesamte Länge gegen Drehen und parallel dazu auch gegen eine Axialverstellung sicherbar sind, wird durch Lösen nur des jeweiligen Aufnahmeteils nicht auch gleichzeitig das Kugelgelenk in seiner axialen Lage beeinflußt. Soll das jeweilige Kugelgelenk jedoch nur in Axialrichtung verschoben werden, so gewährleistet die Drehsicherung, daß keine Verschwenkung der Knochenschrauben erfolgt, sondern diese ihre Winkellage beibehalten. Diese Trennung der einzelnen Freiheitsgrade kann aufgrund der Querschnittsform des Tragkörpers auf der gesamten Länge des Fixateurs und für beide Feststellvorrichtungen gewährleistet werden. Beide Knochenschrauben-Aufnahmeteile können somit hinsichtlich ihrer axialen bzw. drehbaren Fixierung an jeder beliebigen Position des Tragkörpers, auf der gesamten Länge zum Einsatz kommen. Da die Einrichtung aus zwei allseitig verstellbar angeordneten Kugelgelenken besteht, ist eine optimale Anpassung der Knochenschrauben auch bei sehr komplizierten Frakturen ohne weiteres möglich. Diese Anordnung gewährleistet eine sekundenschnelle Reposition axial und/oder in Umfangsrichtung, wobei die Positionen der Feststellvorrichtungen auf dem Tragkörper über die gesamte Länge frei wählbar sind.

Hierzu ist es von Vorteil, daß der Tragkörper im Querschnitt oval-, dreieck- oder sechskantförmig ausgebildet ist.

Ferner ist es von Vorteil, daß ein Ansatz mit einer oder mehreren Feststellschrauben versehen ist, wobei zwischen zwei Verbindungselementen bzw. Kugelgelenkbacken eine Stelleinrichtung bzw. ein Teleskopgestänge vorgesehen ist, über die die Kugelgelenkbacken auf dem Tragkörper axial verschiebbar sind.

Hierzu ist es vorteilhaft, daß der als Gestänge ausgebildete Tragkörper und das Kugelgelenk als Drehsicherung mindestens eine Anlagefläche wie eine Nut zur Aufnahme eines Zwischenglieds wie eine Feder aufweisen, damit ein Verstellen des Kugelgelenks in Axialrichtung des Gestänges unter Gewährleistung der Drehsicherheit zugelassen wird.

Vorteilhaft ist auch, daß das Zwischenglied mindestens an seinem einen Ende ein Feststellelement aufweist, das zwischen dem Kugelgelenk und dem Gestänge eine Klemmverbindung herstellt. Durch die einfache Axialverstellung des Kugelgelenks auf dem Gestänge ist die Längendynamisierung individuell dosierbar. Ferner wird durch die Verwendung von Kugelgelenken der Schwenkradius der einzelnen Knochenschrauben nicht beschränkt, so daß eine größtmögliche Flexibilität in der Positionierung der Knochenschrauben gewährleistet wird.

Von besonderer Bedeutung ist für die vorliegende Erfindung, daß das Zwischenglied an seinen beiden gegenüberliegenden Enden je ein Feststellelement aufweist, das zwischen dem Kugelgelenk und dem Gestänge eine Klemmverbindung herstellt, und daß das Feststellelement des Zwischenglieds aus einem Ringteil gebildet ist, das auf das Gestänge und das Zwischenglied axial aufbringbar ist.

Außerdem ist es vorteilhaft, daß das Feststellelement des Zwischenglieds eine Bohrung zur Aufnahme einer Feststellschraube aufweist, die ein am Zwischenglied fest angeordnetes Klemmteil gegen die Oberfläche des Gestänges preßt und das Kugelgelenk gegen axiales Verschieben oder Drehen sichert.

Hierzu ist es vorteilhaft, daß das Klemmteil an den beiden gegenüberliegenden Enden des Zwischenglieds fest angeordnet ist. Da das Zwischenglied zur Aufnahme der Knochenschrauben mit je einem Feststellelement ausgestattet ist, läßt sich vor Festsetzung des Feststellelements die Knochenschrauben-Klemmbacke mittels des Verbindungselements auf dem Kugelgelenk in jeder gewünschten Position sichern, so daß hierdurch eine optimale Anpassung der Knochenschrauben an die einzelnen unterschiedlich positionierten Knochenteile möglich ist.

Ferner ist es vorteilhaft, daß das Klemmteil eine ebene, gegen die Oberfläche des Gestänges andrückbare, plane Klemmfläche und eine Vertiefung zur Aufnahme der Feststellschraube aufweist.

In weiterer Ausgestaltung der Erfindung ist es vorteilhaft, daß das Klemmteil und/oder ein weiteres Stellelement in seiner Bohrung mit einem Gewinde versehen ist, das auf ein Gewinde des Gestänges aufschraubbar ist. Da die Klemmfläche des Klemmteils gegen die Oberfläche des Gestänges anpreßbar ist, wird die Flächenpressung des Gestänges gering gehalten und dadurch weitgehend Kerbwirkung am Klemmteil vermieden, so daß dieses ohne Beschädigung langfristig einsetzbar ist. Mittels der einzelnen in die Bohrung des Feststellelements bzw. des Ringteils einschraubbaren Feststellschrauben, die über das Klemmteil auf die Oberfläche des Gestänges wirken, wird ebenfalls sichergestellt, daß keine Beschädigungen an der Oberfläche des Gestänges auftreten.

Außerdem ist es vorteilhaft, daß dem Stellelement ein Meßteil zugeordnet ist und daß das Stellelement gegen das Kugelgelenk bzw. das dem Kugelgelenk zugeordnete Feststellelement anpreßbar ist. Durch die Verwendung des gegen die Oberfläche des Kugelgelenks bzw. des Feststellelements anpreßbaren Stellelements läßt sich das Kugelgelenk bei Distraktions- bzw. Kompressionsmaßnahmen millimetergenau verstellen. Beispielsweise ist es möglich, daß der Patient nach der Operation den Fixateur selbst bedient. Hierzu wird durch Umdrehung des Stellelements am Gestänge beispielsweise eine 1 mm-Distraktion bewirkt. Durch die hohe Stabilität des erfindungsgemäßen Fixateurs kann der Patient schon nach kurzer Zeit das betroffene Bein problemlos leichten Belastungen aussetzen.

Mit dem Fixateur können ferner Pseudarthrosen komprimiert und distrahiert werden. Hierdurch wird ein unilaterales Fragmentshifting auch über große Strecken möglich. Ferner bietet der Fixateur durch die universelle Anpassung der Kugelgelenke die Möglichkeit der simultanen Achskorrektur und des simultanen Längenausgleichs.

Hierzu ist es vorteilhaft, daß das Kugelgelenk eine ballige Außenoberfläche aufweist, auf der ein die Knochenschrauben aufnehmendes Verbindungselement verstellbar und allseitig verschwenkbar gelagert ist.

Von Vorteil ist es ferner, daß das Verbindungselement ringförmig ausgebildet und mindestens auf der einen Seite geschlitzt bzw. geteilt ist und zwei mit Abstand zueinander angeordnete Klemmbacken mit koaxial zueinander ausgerichteten Bohrungen aufweist, von denen zumindest die eine Bohrung als Gewindebohrung zur Aufnahme einer Klemmschraube ausgebildet ist, und daß auf der den Klemmbacken gegenüberliegenden Seite ein Tragteil zur Aufnahme des Knochenschrauben-Aufnahmeteils vorgesehen ist. Durch die Verwendung der Klemmbacken des Verbindungselements ist nach allseitiger Verstellung des Verbindungselements auf dem Kugelgelenk eine schnelle Fixierung bzw. Arretierung der Knochenschrauben-Klemmbacken in kürzester Zeit und ohne großen Aufwand möglich.

Eine wesentliche, vorteilhafte Ausführungsform erreicht man dadurch, daß das Knochenschrauben-Aufnahmeteil auf dem Tragteil drehfest gelagert ist.

In weiterer Ausgestaltung der Erfindung ist es vorteilhaft, daß das Knochenschrauben-Aufnahmeteil auf dem Tragteil drehbar gelagert und in verschiedenen Positionen feststellbar ist. Durch die Drehlagerung des Knochenschrauben-Aufnahmeteils auf dem Kugelgelenk ist eine zusätzliche Anpassung der einzelnen Knochenschrauben möglich, wenn dies je nach Ausführungsform des Fixateurs gewünscht wird.

Ferner ist es vorteilhaft, daß jedes Knochenschrauben-Aufnahmeteil eine oder mehrere Knochenschrauben trägt und daß das Klemmbackenteil bzw. das Knochenschrauben-Aufnahmeteil eine Öffnung oder eine Bohrung aufweist, in die ein Ansatz des Knochenschrauben-Aufnahmeteils bzw. des Verbindungselements einsetzbar und sicherbar ist.

Außerdem ist es vorteilhaft, daß der zylindrische Ansatz als Drehlager und als Festlager ausgebildet ist. Soll beispielsweise die Knochenschrauben-Klemmbacke auf dem zylindrischen Ansatz gedreht werden, so kann dieser als Drehlager ausgebildet sein und ferner die Möglichkeit bieten, die Knochenschrauben-Klemmbacken auf dem Drehlager zu arretieren. Ferner besteht auch die Möglichkeit, den zylindrischen Ansatz des Verbindungselements als Festlager auszubilden, so daß die Knochenschrauben-Klemmbacken gegenüber dem Ansatz nicht weiter verstellbar sind.

Besonders vorteilhaft ist es, daß ein Teil des Zwischenglieds, der in der Nut aufgenommen oder gegenüber der Anlagefläche vorgesehen ist, im eingebauten Zustand des Zwischenglieds einen Abstand zu einer Oberfläche der Nut oder der Anlagefläche aufweist und daß die Klemmfläche des Zwischenglieds außerhalb der Bohrung des Kugelgelenks vorgesehen ist.

Ferner ist es vorteilhaft, daß die Klemmfläche kleiner ist als die übrige sich an die Klemmfläche anschließende Oberfläche des Zwischenglieds, das mit Abstand zur Oberfläche der Nut angeordnet ist, wenn die Feststellvorrichtung auf dem Tragkörper aufsitzt, und daß das Gestänge und/oder die Knochenschrauben längenveränderlich ausgebildet sind.

Durch den zwischen der Oberfläche der Nut und der gegenüberliegenden Oberfläche des Zwischenglieds gebildeten Spalt wird auch bei hoher Paßgenauigkeit eine gute Verstellmöglichkeit der Verstelleinrichtung gewährleistet, selbst dann, wenn der Tragkörper Biegebelastungen ausgesetzt ist. Für die Feststellung des Kugelgelenks genügt also eine kleine Klemmfläche, während der übrige Teil der innenliegenden Oberfläche des Zwischenglieds berührungsfrei in der Nut aufsitzt, also nicht als Klemmfläche fungiert. Dabei ist es vorteilhaft, daß die Klemmfläche des Zwischenglieds außerhalb der Bohrung des Kugelgelenks liegt.

Außerdem ist es vorteilhaft, daß zumindest eine Kugelgelenkbacke aus zwei Halbschalen besteht, die auf der einen Seite über ein Gelenk und auf der anderen Seite über eine Klemmschraube miteinander verbunden sind. Hierdurch läßt sich die Kugelgelenkbacke seitlich auf den Tragkörper aufsetzen, auch dann, wenn sich der Fixateur bereits im Einsatz befindet und ein axiales Aufbringen der Kugelgelenkbacke nicht mehr möglich ist.

Eine zusätzliche Möglichkeit ist gemäß einer Weiterbildung der erfindungsgemäßen Vorrichtung, daß die das Kugelgelenk aufweisende Feststellvorrichtung zwei oder mehrere Feststellschrauben aufweist. Dadurch lassen sich die Feststellschrauben auch für andere Aufgaben einsetzen, beispielsweise um zusätzlich an den Feststellschrauben einen Verlängerer anzusetzen.

In weiterer Ausgestaltung der Erfindung ist es vorteilhaft, daß an das Kugelgelenk ein Ansatz zur Aufnahme des Arretierteils bzw. der Feststellschraube einteilig angeordnet ist. Hierdurch wird die Handhabung zur Verstellung des Kugelgelenks weiter verbessert.

Gemäß einer bevorzugten Ausführungsform der erfindungsgemäßen Lösung ist schließlich vorgesehen, daß beiderseits des Kugelgelenks je ein Ansatz zur Aufnahme mindestens je eines Arretierteils bzw. mindestens je einer Feststellschraube einteilig angeordnet ist. Hierdurch kann die eine Feststellschraube zum Festsetzen des Ansatzes auf der Tragvorrichtung und der zweite Ansatz mit der zugehörigen Feststellschraube zur Aufnahme des Verlängerers eingesetzt werden.

Von besonderer Bedeutung ist für die vorliegende Erfindung, daß der Ansatz mit einer oder mehreren Feststellschrauben versehen ist.

Im Zusammenhang mit der erfindungsgemäßen Ausbildung und Anordnung ist es von Vorteil, daß zwischen zwei Verbindungselementen bzw. Kugelgelenkbacken eine Stelleinrichtung bzw. ein Teleskopgestänge vorgesehen ist, über die die Kugelgelenkbacken auf dem Tragkörper axial verschiebbar sind, so daß das Teleskopgestänge als Extensions oder Kontraktionsgerät eingesetzt werden kan.

Vorteilhaft ist es ferner, daß das Teleskopgestänge über an den Kugelgelenkbacken vorgesehene Klemmvorrichtungen festsetzbar ist.

Außerdem ist es vorteilhaft, daß das Teleskopgestänge aus einer ein Gewinde aufweisenden Hülse und einem ebenfalls ein Gewinde aufweisenden Gestänge besteht.

Hierzu ist es vorteilhaft, daß der Tragkörper im Querschnitt kreisförmig, ovalförmig oder sechskantförmig ausgebildet ist und mindestens ein Drehsicherungsteil aufweist, das ein Drehen des Kugelgelenks verhindert und eine Axialverstellung auf der Tragvorrichtung gestattet.

Ferner ist es vorteilhaft, daß der Tragkörper aus Kohlefaser gebildet ist, die aus einzelnen, auf einem Kern spiralförmig aufwickelbaren Kohlefasersträngen besteht, die nach dem Aufwickeln auf den Kern zu einer bleibenden Querschnittsform verpreßt werden.

Eine zusätzliche Möglichkeit ist gemäß einer Weiterbildung der erfindungsgemäßen Vorrichtung, daß das Kugelgelenk aus zwei mittig teilbaren Lagerschalenhälften besteht, auf die die Kugelgelenkbacken radial aufbringbar sind.

Auf diese Weise wird erreicht, daß die eine Lagerschalenhälfte einen ersten gegen die Außenseite der Kugelgelenkbacke anlegbaren Flansch und die zweite Lagerschalenhälfte einen zweiten nach der Montage der Lagerschale in eine Anlageposition gegen die Außenseite der Kugelgelenkbacke bringbaren Flansch aufweist.

Vorteilhaft ist es auch, daß die Lagerschale des Verbindungselements einen Ausschnitt in Größe der Breite des Kugelgelenks aufweist, so daß durch Drehen des Kugelgelenks um seine Achse um 90° das Kugelgelenk in die Lagerschale einsetzbar und durch nochmaliges Drehen um 90° sicherbar ist. Hierzu ist es von Bedeutung, daß die Lagerschale seitlich keine Ansätze aufweist und daß die Breite des Kugelgelenkstücks etwas kleiner ist als die Breite des Ausschnitts.

Weitere Vorteile und Einzelheiten der Erfindung sind in den Patentansprüchen und in der Beschreibung erläutert und in den Figuren dargestellt, wobei bemerkt wird, daß alle Einzelmerkmale und alle Kombinationen von Einzelmerkmalen erfindungswesentlich sind. Es zeigt:
- Figur 1a und 1b: eine Schnittdarstellung von zwei Ausführungsbeispielen eines Fixateurs mit zwei auf einem Gestänge angeordneten Kugelgelenken zur allseitig verschwenkbaren Aufnahme je eines Verbindungselements mit Knochenschrauben-Klemmbacken,
- Figur 2: eine Teilansicht des Kugelgelenks mit dem zugehörigen Verbindungselement zur Aufnahme einer Knochenschrauben-Klemmbacke,
- Figur 3: eine Vorderansicht des Kugelgelenks gemäß Figur 2,
- Figur 4: eine Ansicht des Kugelgelenks und des zugehörigen, als Feder ausgebildeten Zwischenglieds bzw. der Feststellvorrichtung in der Ansicht von unten gemäß Figur 5,
- Figur 5: eine Schnittdarstellung des Kugelgelenks sowie eines Teils des Gestänges mit der zugehörigen Feststellvorrichtung,
- Figur 6: eine Schnittdarstellung entlang der Linie 5-5 gemäß Figur 5,
- Figur 7: eine Schnittdarstellung eines zweiten Ausführungsbeispiels des Kugelgelenks mit zwei fest angeschlossenen, zylindrischen Ansätzen zur Aufnahme von Feststellschrauben,
- Figur 8: eine Schnittdarstellung des zweiten Ausführungsbeispiels des Kugelgelenks mit einem fest angeschlossenen, zylindrischen Ansatz zur Aufnahme von einer oder mehreren Feststellschrauben,
- Figur 9: eine Seitenansicht eines ein Gelenk aufweisenden Feststellelements mit einem aus zwei Teilen gebildeten Kugelgelenk in geöffneter Stellung,
- Figur 10: eine Seitenansicht eines ein Gelenk aufweisenden Feststellelements mit einem aus zwei Teilen gebildeten Kugelgelenk in geschlossener Stellung,
- Figur 11: eine Seitenansicht eines ein Gelenk aufweisenden Feststellelements mit Klemmbackenteilen zur Aufnahme von Knochenschrauben ähnlich wie in Figur 2,
- Figur 12: eine Draufsicht gemäß Figur 11,
- Figur 13a und 13b: eine Schnittdarstellung einer auf das Kugelgelenk aufbringbaren Lagerschale und eines Teils des Verbindungselements gemäß Figur 2,
- Figur 14 und 15: zwei verschiedene Klemmvorrichtungen für einen Verlängerer,
- Figur 16: einen Querschnitt des Tragkörpers gemäß Figur 1b,
- Figur 17: einen Längsschnitt des Tragkörpers gemäß Figur 1b,
- Figur 18: eine Vorderansicht der Lagerschale des Kugelgelenks gemäß Figur 8,
- Figur 19: eine Seitenansicht der Lagerschale des Kugelgelenks gemäß Figur 8 und 18.

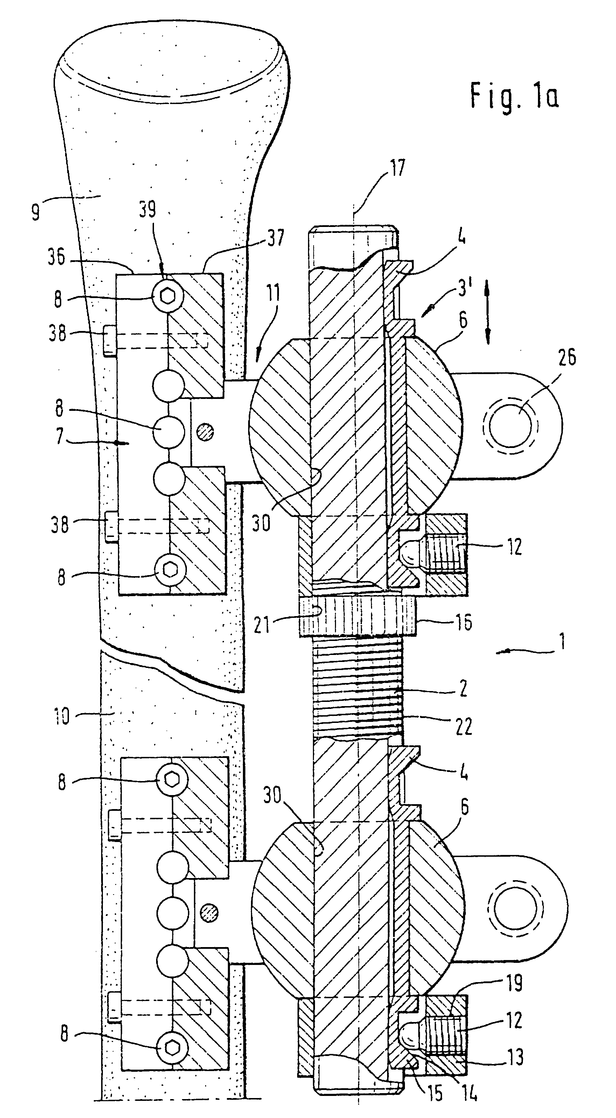

In der Zeichnung ist in Figur 1a mit 1 ein Fixateur bezeichnet, der bei der Frakturversorgung bzw. bei Fragmentshifting bzw. Distraktions- und Kompressionsmaßnahmen einsetzbar ist. Der Fixateur 1 besteht aus einem Tragkörper 2, der als Gestänge ausgebildet ist und über einen Teil des Gestänges bzw. über die gesamte Länge des Gestänges mit einem Gewinde 22 versehen sein kann. Das Gestänge 2 kann aus Verbundwerkstoff, in vorteilhafter Weise aus einem Kohlefaserwerkstoff, bestehen, so daß es bei Röntgenaufnahmen weitgehend unsichtbar bleibt. Es ist auch möglich, das Gestänge aus einer Metallegierung herzustellen. Das Gestänge 2 ist normalerweise ohne Gewinde 22 versehen und weist daher eine glatte Oberfläche auf, damit eine einwandfreie bzw. ruckfreie Verstellung der zu verstellenden Teile möglich ist. Sollte jedoch das Gewinde 22 verwendet werden, so ist die Oberfläche des Gewindes so behandelt, daß die Gleiteigenschaften nicht beeinträchtigt werden.

Der Tragkörper bzw. das Gestänge 2 weist ferner eine sich in einer Längsachse 17 des Gestänges 2 erstreckende Nut 3 als Anlagefläche auf, die als Drehsicherung des Kugelgelenks 6 dient.

Auf dem Gestänge 2 können zwei oder mehrere mit Abstand zueinander angeordnete Kugelgelenke 6 axial verschiebbar angeordnet sein.

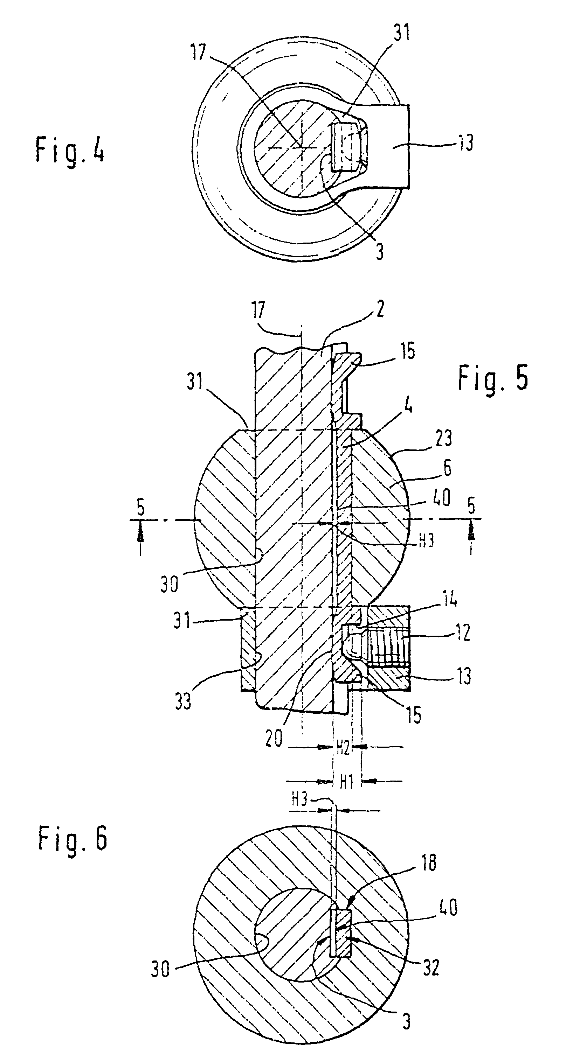

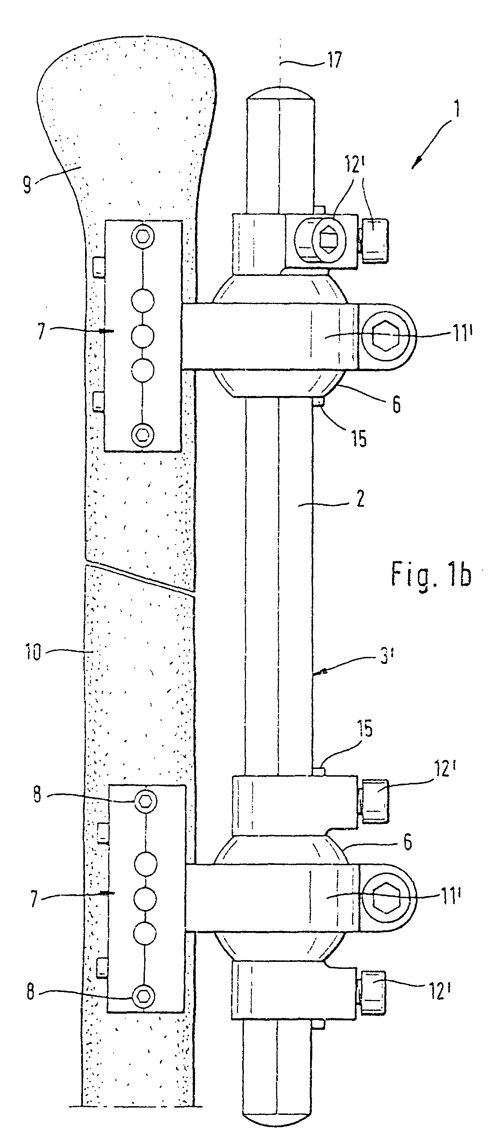

Das Kugelgelenk 6 besteht gemäß Figur 1 und 5 aus einer eine Bohrung 30 aufweisenden Kugel mit einer balligen Außenoberfläche 23, die durch zwei gegenüberliegende, parallel verlaufende, plane Abschlußflächen 31 bzw. Segmentflächen begrenzt wird. Die Segmentfläche 31 schneidet die Längsachse 17 in einem rechten Winkel und ist, wie aus Figur 6 hervorgeht, kreisförmig ausgebildet.

Das Kugelgelenk 6 ist ebenfalls mit einer Nut 18 ausgestattet, die im Querschnitt in etwa der Nut 3 des Gestänges 2 entspricht. Wird das Kugelgelenk 6 auf das Gestänge 2 aufgeschoben, so können die beiden Nuten 3 und 18 zueinander ausgerichtet werden und bilden dann eine im Querschnitt rechteckförmige Fläche 32. Bevor das Kugelgelenk 6 auf das Gestänge 2 aufgeschoben wird, muß zuerst ein **als Feder ausgebildetes Zwischenglied 4** in die Bohrung 30 des Kugelgelenks 6 und die zugehörige Nut 18 eingeführt werden. Die Feder bzw. das Zwischenglied 4 weist an ihren beiden gegenüberliegenden Enden je ein Klemmteil 15 auf, das gemäß Figur 5 eine Höhe H₁ aufweist, die größer ist als die Höhe H₂ des mittleren Teils des Zwischenglieds 4. Hierdurch kann das Zwischenglied 4 bei entsprechender Paßbildung mit den beiden Klemmteilen 15 durch die Bohrung 30 hindurchgeführt und in die Nut 18 des Kugelgelenks 6 eingesetzt bzw. auch gleichzeitig festgeklemmt werden, wenn entsprechend kleine Toleranzen zwischen den beiden gegenüberliegenden Stirnflächen des Klemmteils 15 eingehalten werden. Dadurch wird die Montage des Kugelgelenks 6 in Verbindung mit der **Feststellvorrichtung** auf dem Gestänge 2 sehr erleichtert. Zwischen der Außenoberfläche des Gestänges 2 und der Innenoberfläche der Nut 3 wird gemäß Figur 1, 3 und 6 ein Zwischenraum gebildet, der mit H₃ gekennzeichnet ist. Hierdurch wird gewährleistet, daß sich das Kugelgelenk 6 mit der Feder 4 auf dem Gestänge auch dann noch ohne weiteres verschieben läßt, wenn sich das Gestänge 2 aufgrund extremer Belastung durchbiegen würde. Der Abstand des Zwischenraums H₃ ist in vorteilhafter Weise zwischen 0,5 und 6 mm bzw. zwischen 1 und 2 mm groß. Nachdem das Zwischenglied 4 in das Kugelgelenk 6 eingesetzt wurde, lassen sich beide Teile auf das Gestänge 2 aufschieben, so daß sie dann eine Position gemäß Figur 1 einnehmen können. Die Verwendung des **Zwischenglieds** 4 hat den großen Vorteil, daß bei hoher Paßgenauigkeit auch eine einwandfreie Verstellung von Kugelgelenk 6 und Zwischenglied 4 möglich ist. Aus diesem Grund liegt das **Zwischenglied** 4 nur mit seinen beiden Zungen 5 bzw. seinen kurzen Klemmflächen 20 in der Nut 3 auf. Das hat, wie bereits erwähnt, den großen Vorteil, daß bei einer geringen Biegung des Tragkörpers 2 kein Klemmen auftritt, wenn das **Zwischenglied** 4 in der Nut 3 verschoben wird. Der mittlere Teil des **Zwischenglieds** 4 weist deshalb einen kleinen Abstand H₁ zur Oberfläche der Nut 3 auf. Hier werden die Gleiteigenschaften des **Zwischenglieds** 4 wesentlich verbessert.

Die Feststellung des Kugelgelenks 6 auf dem Gestänge 2 erfolgt über das eine Klemmteil 15 und ein zugehöriges Feststellelement 13. Es können auch zwei Klemmteile 15 verwendet werden. Das Kugelgelenk 6 ist mittels der Nut 3 rotationsgesichert, d. h. es kann gemäß Figur 1 nur in Richtung der Längsachse 17 verschoben werden.

Das Feststellelement 13 besteht gemäß Figur 5 aus einem ringförmigen Teil mit einer Gewindebohrung zur Aufnahme einer Feststellschraube bzw. Senkkopfschraube 12, die, nachdem sie in die Bohrung des Feststellelements 13 eingeschraubt wurde, in eine Vertiefung 14 eingreift. Die Vertiefung 14 sitzt in dem Klemmteil 15. Durch Eindrehen der Feststellschraube 12 in die Bohrung 19 wird die Klemmfläche 20 des Klemmteils 15 gegen die Oberfläche des Gestänges 2 gepreßt und stellt dadurch eine sehr gute Klemmverbindung zwischen dem Feststellelement 13, dem zugehörigen Kugelgelenk 6 und dem Gestänge 2 her, so daß das Kugelgelenk 6 gegen eine Axialverstellung auf dem Gestänge 2 gesichert ist. Durch die plane Klemmfläche 20 des Klemmteils 15 wird eine gute Flächenpressung und somit Klemmwirkung zwischen dem Feststellelement 13 und dem Gestänge 2 unter Vermeidung von Kerbwirkungen an der Oberfläche des Gestänges 2 erreicht.

Im Ausführungsbeispiel gemäß Figur 5 ist **das Zwischenglied 4** an **seinen** beiden Enden mit je einem Klemmteil 15 ausgestattet. Es ist jedoch auch möglich, nur an einer Seite des Zwischenglieds 4 ein entsprechendes Klemmteil 15 vorzusehen.

Wie aus Figur 5 ferner hervorgeht, deckt eine Bohrung 33 des Feststellelements 13 das zu sichernde Klemmteil 15 vollständig ab, so daß dadurch auch eine Schutzwirkung für das Klemmteil 15 erzielt wird.

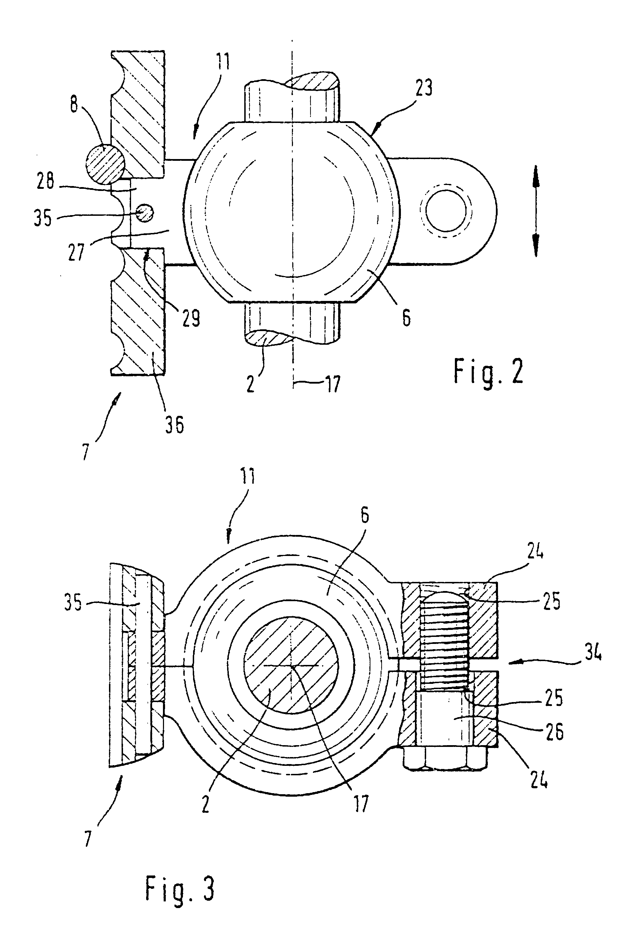

Wie aus den Figuren 2 und 3 hervorgeht, ist die Außenoberfläche 23 des Kugelgelenks 6, wie bereits erwähnt, ballig ausgebildet und dient als Lagerfläche eines Verbindungselements 11. Das Verbindungselement 11 ist ringförmig ausgebildet und an einer Seite mit einer schlitzförmigen Öffnung 34 versehen, so daß hierdurch zwei mit Abstand zueinander angeordnete Klemmbacken 24 gebildet werden, die je eine Bohrung 25 aufweisen. Gemäß Figur 3 kann die obere Klemmbacke 24 mit der Gewindebohrung 25 versehen sein, in die eine Klemmschraube 26 eindrehbar ist, so daß die beiden Klemmbacken 24 zusammengezogen werden und somit das Verbindungselement 11 auf der Außenoberfläche 23 des Kugelgelenks 6 festgesetzt werden kann. Wird beispielsweise die Klemmschraube 26 gelöst, so ist das Verbindungselement 11 auf der Außenoberfläche 23 allseitig schwenkbar, wobei der Schwenkbereich des Verbindungselements 11 durch Anlage des Knochenschrauben-Aufnahmeteils 7 gegen die Oberfläche des Gestänges 2 begrenzt wird. Durch Verstellen des Verbindungselements 11 mit dem zugehörigen Knochenschrauben-Aufnahmeteil 7 können auch entsprechende im Klemmteil 15 aufgenommene Knochenschrauben 8 ebenfalls ohne weiteres allseitig verstellt werden. Hierdurch erhält man eine optimale Anpassung der Knochenschrauben 8 an die entsprechenden Knochenteile, z. B. eines Ober- und/oder Unterschenkels 9, 10, bei einer Frakturversorgung.

Wie aus Figur 2 hervorgeht, befindet sich gegenüber den beiden Klemmbacken 24 ein einen zylindrischen Ansatz 28 aufweisendes Tragteil 27, auf dem das Knochenschrauben-Aufnahmeteil 7 gelagert und mittels eines Bolzens 35 drehgesichert ist.

Je nach Ausführungsform ist es jedoch auch möglich, das Knochenschrauben-Aufnahmeteil 7 auf dem Tragteil 27 drehbar zu lagern, wobei das Knochenschrauben-Aufnahmeteil 7 mittels eines nicht dargestellten Sprengrings auf dem Tragteil 27 gesichert werden kann.

Das Knochenschrauben-Aufnahmeteil 7 besteht gemäß Figur 1 aus zwei gegeneinander anlegbaren Klemmbackenteilen 36 und 37, die über Schraubenbolzen 38 zusammengehalten werden. Die Schraubenbolzen 38 werden hierzu in entsprechende in den Klemmbackenteilen 36 und 37 vorgesehene Bohrungen eingeschraubt. Jedes Klemmbackenteil 36, 37 weist halbkreisförmige Vertiefungen auf, die beim Zusammenfügen der beiden Klemmbackenteile 36 und 37 eine zylindrische Öffnung 39 bilden, in denen die Knochenschrauben 8 verstellbar aufgenommen und mittels der Schraubenbolzen 38 festgeklemmt werden.

Im Ausführungsbeispiel gemäß Figur 1 sind lediglich zwei mit Abstand zueinander angeordnete Kugelgelenke 6 auf dem Gestänge 2 angeordnet. Es ist jedoch auch möglich, bei Mehrfachfrakturen mehrere Kugelgelenke 6 auf dem Gestänge 2 zu plazieren und, wie beschrieben, zu arretieren.

Im Ausführungsbeispiel gemäß Figur 2 ist eine Bohrung 29 des Klemmbackenteils 36 zylinderförmig ausgebildet, insbesondere dann, wenn der Ansatz als Drehlager ausgebildet sein soll. Es ist natürlich auch möglich, das Tragteil 27 und den zugehörigen Ansatz 28 rechteckförmig auszubilden, um auf diese Weise eine Drehsicherung zwischen dem Tragteil 27 und dem Knochenschrauben-Aufnahmeteil 7 herzustellen.

Gemäß Figur 1 kann neben dem Feststellelement 13 ein weiteres mit einer Bohrung 25 versehenes Stellelement 16 vorgesehen sein, das als Stellschraube für die Feineinstellung des Kugelgelenks 6 auf dem Gestänge 2 eingesetzt werden soll, insbesondere dann, wenn der Fixateur bei Distraktions- bzw. Kompressionsmaßnahmen eingesetzt werden soll. Die Stellschraube 16 kann mit einem eine geringe Steigerung aufweisenden Gewinde 21 ausgestattet sein, so daß sie auf das ein Gewinde 22 aufweisende Gestänge 2 aufgeschraubt werden kann, so daß beispielsweise bei einer Umdrehung der Stellschraube 16 eine Verstellung des Kugelelements auf der Stange 2 um 1 mm erfolgt. Hierdurch ist es möglich, daß beispielsweise nach einer Operation der Patient den Fixateur selbst bedienen kann, um die Distraktion zu beeinflussen. Er kann auf einfache Weise die Stellschraube jeweils um 1 mm pro Tag verstellen.

Der in der Anmeldung bezeichnete Fixateur 1 kann als Haltevorrichtung oder Außenspanner für die Behandlung von Knochenerkrankungen bzw. Knochenbrüchen bezeichnet werden. Ferner kann es besonders vorteilhaft sein, wenn der Außenspanner auch als einseitiger (gemäß Figur 1) oder auch als zweiseitiger Außenspanner ausgebildet ist. Besonders vorteilhaft ist es auch, daß das Gestänge 2 und/oder die Knochenschrauben 8 längenveränderlich ausgebildet sind.

In der Zeichnung ist in Figur 1b mit 1 ein zweites Ausführungsbeispiel eines Fixateurs bezeichnet, der bei der Frakturversorgung bzw. bei Fragmentshifting bzw. Distraktions- und Kompressionsmaßnahmen einsetzbar ist.

Der Fixateur 1 besteht aus dem Tragkörper 2, der als Gestänge ausgebildet ist und über die gesamte Länge des Gestänges im Querschnitt entweder oval, dreieckförmig, rechteckförmig oder insbesondere sechskantförmig ausgebildet sein kann. Auf jeden Fall soll der Querschnitt so ausgebildet sein, daß das Kugelgelenk gemäß Figur 1a, 1b, 7 und 8 sich auf dem Tragkörper 2 nicht drehen kann, wenn eine der Feststellschrauben 12, 12' oder 26 gelöst wird.

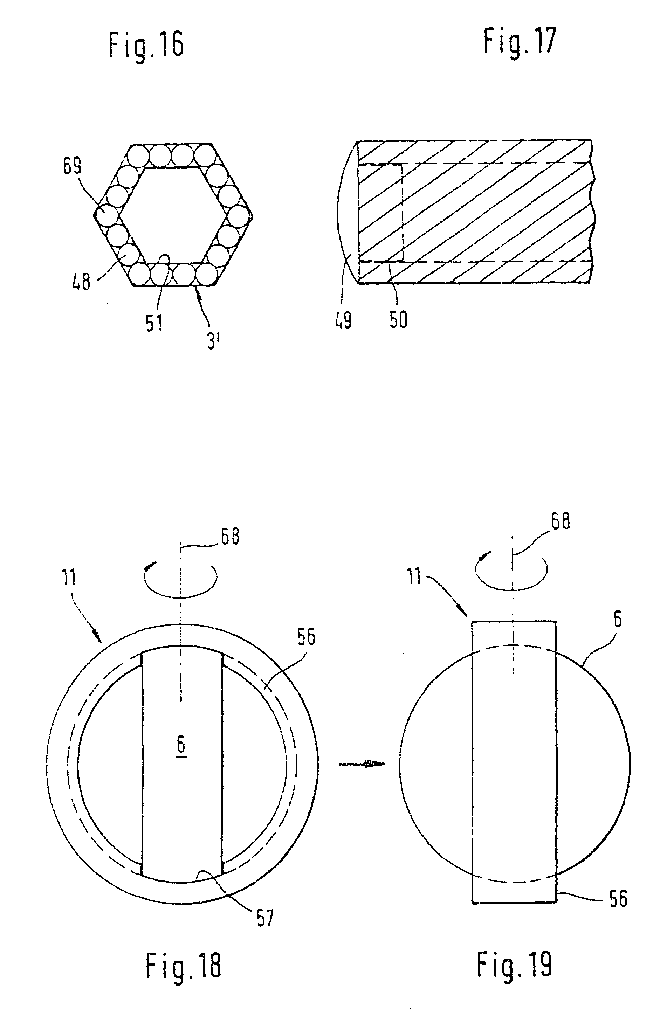

Hierzu weist der Tragkörper 2 **eine als Drehsicherung** für das Kugelgelenk 6 ausgebildete Anlagefläche 3 auf, die entweder gemäß Figur 1a als Nut 3 (vgl. Figur 1a) oder als **sechseckige** Anlagefläche 3 (vgl. Figur 16) ausgebildet ist.

Das Gestänge 2 kann aus Verbundwerkstoff, in vorteilhafter Weise aus einem Kohlefaserwerkstoff, bestehen, so daß es bei Röntgenaufnahmen weitgehend unsichtbar bleibt. Es ist auch möglich, das Gestänge aus einer Metallegierung oder aus einem nicht rostenden Metall, beispielsweise aus Titan, oder aus Gewichtsgründen aus Al herzustellen. Das Gestänge 2 ist gemäß Figur 1b ohne Gewinde 22 ausgebildet und weist daher eine glatte Oberfläche mit mindestens einer Anlagefläche 3 auf, damit eine einwandfreie bzw. ruckfreie Verstellung der zu verstellenden Teile möglich ist.

Der Tragkörper 2 besteht aus einem Sechskantkörper (Figur 16), der aus Kohlefaser gebildet ist und aus einzelnen Kohlefasersträngen 48 hergestellt wird.

Die Kohlefaserstränge 48 werden zur Herstellung des Gestänges 2 auf einem in der Zeichnung nicht dargestellten Kern, der nur für den Wickelvorgang benötigt wird, spiralförmig aufgewickelt, die nach dem Aufwickelvorgang zu einer bleibenden Querschnittsform verpreßt werden und dann von dem nicht dargestellten Kern abgezogen werden. Hierdurch wird sichergestellt, daß die einzelnen Stränge 69 an der Oberfläche nicht beschädigt bzw. angeschnitten werden, wenn die Sechskantform fertiggestellt wird.

Der Tragkörper bzw. das Gestänge 2 weist zumindest eine Anlagefläche 3 und gemäß Figur 16 sechs sich in Richtung der Längsachse 17 (Figur 1a) erstreckende Anlageflächen auf, die einzeln oder gemeinsam als Drehsicherung für das Kugelgelenk 6 dienen, wenn eine der Stellschrauben 12', 26 gelöst wird.

Auf dem Gestänge 2 können zwei oder mehrere mit Abstand zueinander angeordnete Kugelgelenke 6 gemäß Figur 1a, 1b oder Figur 8 bis 10 axial verschiebbar angeordnet sein.

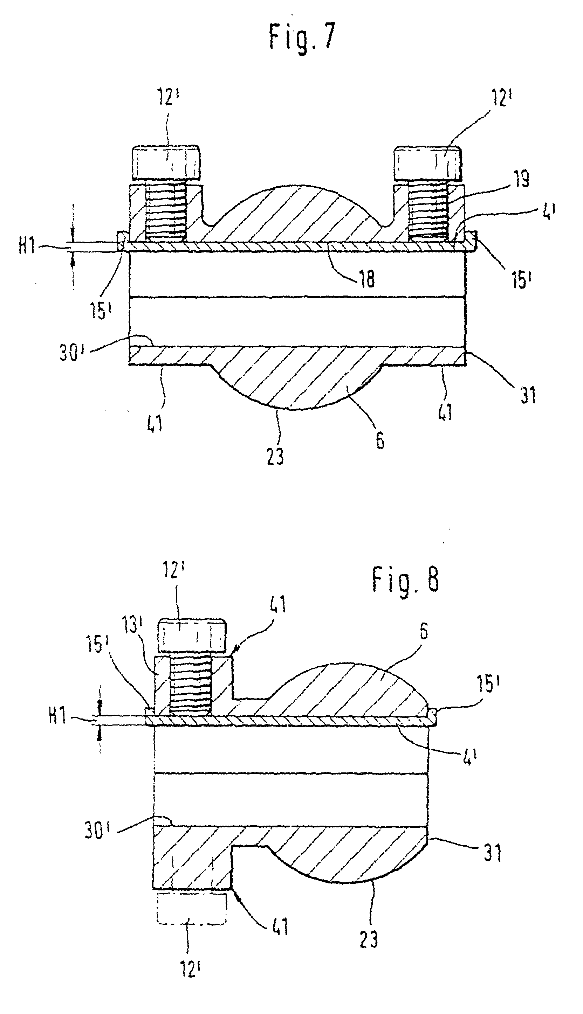

Das Kugelgelenk 6 besteht gemäß Figur 8 aus einer eine Sechskant-Bohrung 30' aufweisenden Kugel 6 mit einer balligen Außenoberfläche 23, die durch zwei gegenüberliegende, parallel verlaufende Ansätze 41 (Figur 7) begrenzt wird, die zylinderförmig ausgebildet sein können oder auch eine andere Querschnittsfläche, ähnlich dem Tragkörper 2, aufweisen können.

Gemäß Figur 8 ist an das Kugelgelenk 6 nur ein Ansatz 41 zur Aufnahme des Arretierteils bzw. der Feststellschraube 12' einteilig angeordnet.

Soll, wie später noch erläutert (Figur 18, 19), das Kugelgelenk 6 seitlich in das Verbindungselement 11 eingeschoben werden, so kann der Ansatz 41 auch mit dem Kugelgelenk 6 lösbar verschraubt, verklemmt oder über zusätzliche Verbindungselemente verbunden sein.

Die Ansätze 41 dienen gemäß Figur 7 und Figur 8 zur Aufnahme mindestens einer oder zweier oder mehrerer Feststellschrauben 12'.

Das Kugelgelenk 6 ist ebenfalls mit einer Nut 18 ausgestattet, die im Querschnitt in etwa der Nut bzw. der Anlagefläche 3 des Gestänges 2 entspricht. Wird das Kugelgelenk 6 auf das Gestänge 2 aufgeschoben, so können die beiden Anlageflächen 3 und 18 zueinander ausgerichtet werden und bilden dann eine im Querschnitt rechteckförmige Fläche. Bevor das Kugelgelenk 6 auf das Gestänge 2 aufgeschoben wird, muß zuerst ein als Feder **ausgebildetes Zwischenglied 4** in die Bohrung 30' des Kugelgelenks 6 eingeführt werden. Die Feder bzw. das Zwischenglied 4 weist an ihren beiden gegenüberliegenden Enden je ein Hakenteil 15' auf, das gemäß Figur 8 gegen die Fläche 31 des Kugelgelenks 6 zur Anlage gebracht wird, wenn dieses in die Bohrung 30' eingesetzt wird.

Der mittlere Teil der **Feder 4** kann ebenfalls einen kleinen Abstand H₃ zur Oberfläche der Anlagefläche 3, ähnlich wie in Figur 6, aufweisen. Hierdurch werden die Gleiteigenschaften der **Feder 4** wesentlich verbessert.

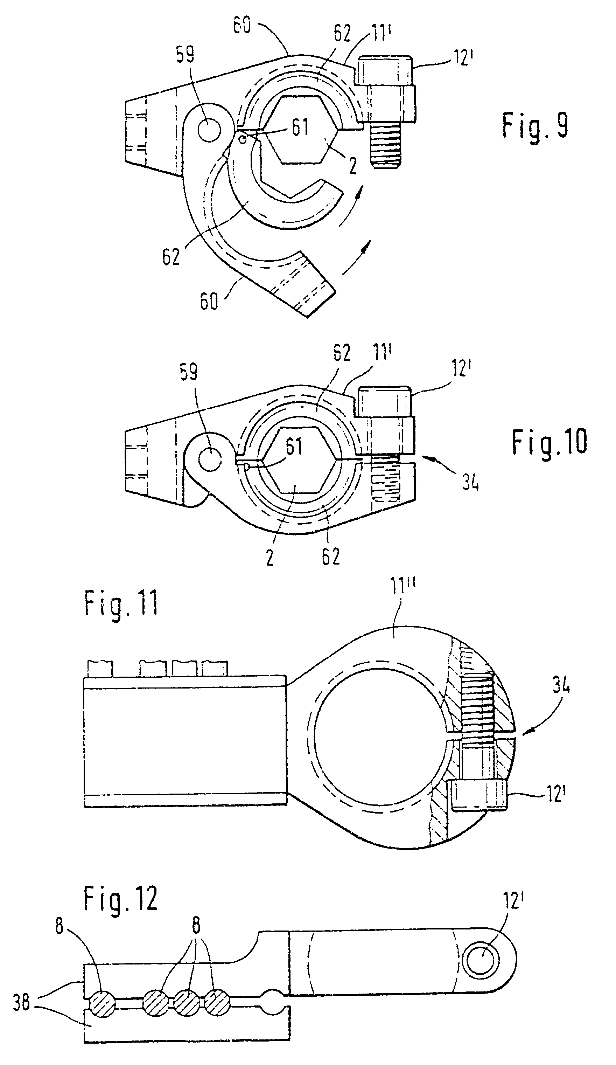

Das in Figur 9 bis 12 dargestellte Verbindungselement 11' und 11" ist ähnlich wie in Figur 3 zumindest an einer Seite geteilt und mit einer schlitzförmigen Öffnung 34 versehen.

Das in Figur 9 und 10 dargestellte Verbindungselement 11' besteht aus zwei über einen Gelenkbolzen 59 miteinander verbundenen Lagerschalen 60, die ebenfalls zusammenfügbar und über die Feststellschraube 12' fest miteinander verbindbar sind. Auf diese Weise ist es möglich, das Verbindungselement 11' noch nachträglich seitlich auf den Tragkörper 2 aufzubringen. Hierzu ist auch die Gelenkkugel 6 aus zwei über einen Gelenkbolzen 61 verbundenen Gelenkkugelteilen 62 gebildet, die in der Stellung gemäß Figur 9 auf den Tragkörper 2 seitlich aufgebracht und dann mittels dem Verbindungselement 11' und der Feststellschraube 12' gesichert werden können.

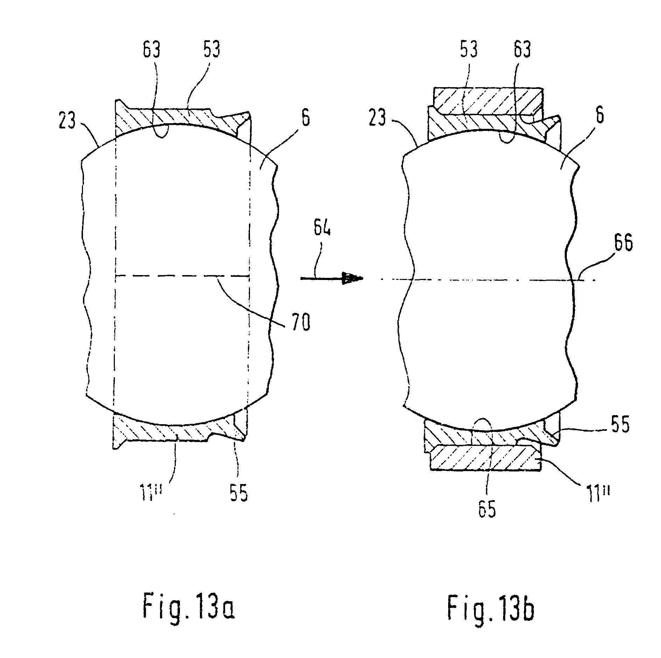

Wie aus Figur 13a und 13b hervorgeht, kann auf die Außenoberfläche 23 des Kugelgelenks 6 eine Lagerschale 53 aufgesetzt werden, deren Innenoberfläche 63 der Außenoberfläche 23 des Kugelgelenks 6 angepaßt ist. Die Lagerschale 53 kann bei 70 mittig geteilt sein und nach Aufsetzen der Lagerschalen 53 zusammengefügt werden. Wenn die beiden Lagerschalen 53 gemäß Figur 13b auf der Gelenkkugel 6 gesichert worden sind, werden diese gemeinsam gemäß Pfeil 64 in die Bohrung 65 des Verbindungselements 11'' eingeführt, bis der Flansch 54 gegen die Stirnfläche des Verbindungselements 11'' zur Anlage kommt. Dann wird der zweite Flansch 55, der mit Bezug auf die Mittelachse 66 einen Winkel von 30° bis 60°, insbesondere 45°, bildet, umgebörtelt, so daß er ebenfalls ganzflächig gegen die Stirnseite des Lagerelements 11'' zur Anlage kommt und auf diese Weise die Gelenkkugel 6 in der Bohrung 65 des Verbindungselements 11'' sichert.

Es ist auch möglich, die Lagerschale als einteiliges Stück und nicht bleibend verformbar herzustellen und so über die Gelenkkugel 6 zu pressen.

Gemäß einem weiteren Ausführungsbeispiel gemäß Figur 18 und 19 kann die Lagerschale 56 des Verbindungselements 11 einen Ausschnitt 57 in Größe der Breite des Kugelgelenks 6 aufweisen, so daß durch Drehen des Kugelgelenks 6 um seine Mittelachse 68 um 90° das Kugelgelenk 6 mittels des Ausschnitts 57 seitlich in die Lagerschale 56 einsetzbar und durch nochmaliges Drehen um 90° gemäß Figur 18 in dem Ringraum der Lagerschale 56 sicherbar ist.

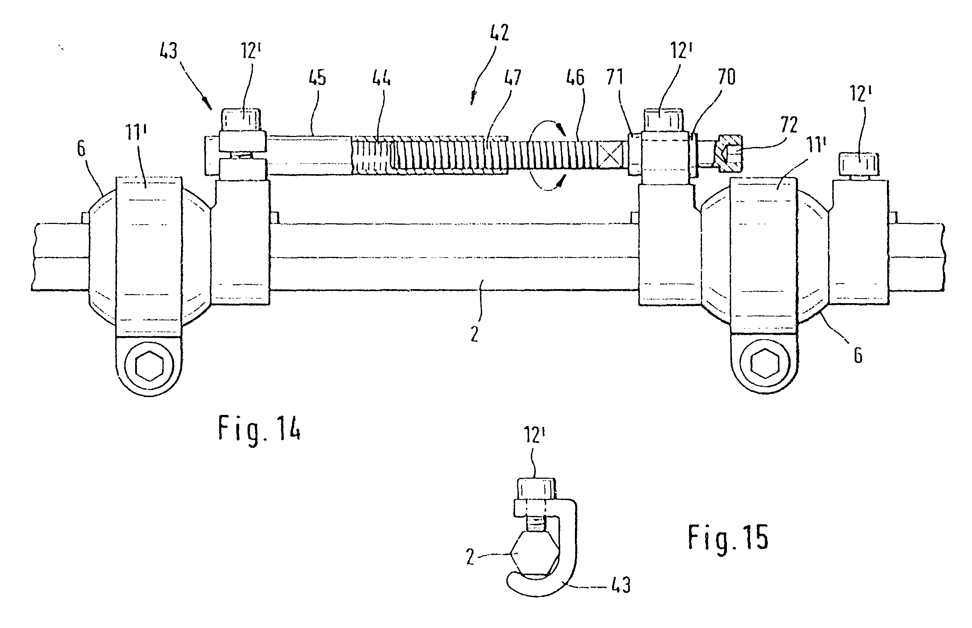

Zwischen zwei Verbindungselementen 11 bzw. 11' kann gemäß Figur 1b und 15 eine Stelleinrichtung bzw. ein Teleskopgestänge 42 vorgesehen sein, das aus einer ein Innengewinde 44 aufweisenden Hülse 45 und einem ebenfalls ein Gewinde 47 aufweisenden Gestänge 46 besteht, die durch Drehen eine Abstandsveränderung zwischen den Feststellschrauben 12' bzw. den Verbindungselementen 11 herbeiführt.

Gemäß Figur 14 und 15 ist das Gestänge 42 in einer Klemmvorrichtung 43 aufgenommen, die mittels der Stellschraube 12' verstellt wird und dadurch das Gestänge 46 sichert bzw. festklemmt oder freigibt, wenn eine Abstandsveränderung zwischen zwei Fixpunkten, z. B. den Verbindungselementen 11, erreicht werden soll.

Gemäß Figur 14 kann das eine Ende des Teleskopgestänges 42 ein Drehlager 70 aufweisen, das mittels zweier, beiderseits gegen das Verbindungselement 11 anliegende Sicherungsringe 71 auf dem Teleskopgestänge 42 in Axialrichtung gesichert ist, jedoch eine Drehung zumindest des einen Teils bzw. des Gestänges 46 zuläßt, so daß durch Drehen des Gestänges 46 die Hülse 45 in Axialrichtung auf dem Gestänge 46 verstellt werden kann und dadurch eine Abstandsveränderung zwischen zwei Kugelgelenken 6 herbeiführt.

Es ist auch möglich, daß anstelle des linken Sicherungsringes ein ein Gewinde aufweisender Stellring oder eine Kontermutter 71 vorgesehen sein kann, die über einen am Ende des Gestänges 46 vorgesehenen, drehbaren Schraubenkopf 72 eine Verstellung des einen Kugelgelenkes 6 gegenüber dem anderen Kugelgelenk 6 herbeiführt. Der Schraubenkopf 72 ersetzt dann den zweiten rechten Sicherungsring. Das rechte Ende des Gestänges 46 lagert in einer in dem Ansatz 41 vorgesehenen Bohrung.

Die Fig 15 dargestellte Klemmvorrichtung 43 wird dann eingesetzt, wenn z. B. das linke Kugelgelenk 6 gemäß Fig 14 wegfällt. In diesem Fall kann sich das rechte Ende über die Klemmvorrichtung 43 auf der Tragvorrichtung 2 direkt abstützen. Das eine Ende des Gestänges 46 wird dann mittels der Schraube 12' und einem nicht dargestellten Lagerauge, ähnlich wie in Fig. 14, an der Klemmvorrichtung 43 befestigt.

### Bezugszeichenliste

- 1: Fixateur, Haltevorrichtung bzw. Außenspanner, einseitig
- 2: Tragkörper, Gestänge
- 3: Anlagefläche, Nut
- 4: Zwischenglied, Feder
- 5: Zunge der Feststellvorrichtung
- 6: Kugelgelenk
- 7: Knochenschrauben-Aufnahmeteil,
Knochenschrauben-Klemmbacke
- 8: Knochenschraube
- 9: Oberschenkel
- 10: Unterschenkel
- 11: Verbindungselement z. Knochenschraube 8 und Fixateur 1
- 11': Verbindungselement, teilbar
- 11'': Verbindungselement, teilbar und mit Gelenk Kugelgelenkbacke
- 12: Arretierteil = Feststellschraube, Senkkopfschraube
- 13: Feststellelement (= Ringteil) für Zwischenglied 4 mit Senkkopfschraube 12
- 14: Vertiefung
- 15: Klemmteil
- 15': Hakenteil
- 16: Stellelement = Stellschraube für Feineinstellung 1mm/Tag
- 17: Längsachse
- 18: Nut
- 19: Bohrung
- 20: Klemmfläche
- 21: Gewinde
- 22: Gewinde
- 23: Außenoberfläche
- 24: Klemmbacke
- 25: Bohrung, Gewindebohrung
- 26: Arretierteil = Klemmschraube
- 27: Tragteil
- 28: Ansatz
- 29: Bohrung
- 30: Bohrung
- 30': Sechskant-Bohrung
- 31: Fläche (Segmentfläche)
- 32: Querschnittsfläche der Nuten 3, 18
- 33: Bohrung
- 34: schlitzförmige Öffnung
- 35: Bolzen
- 36: Klemmbackenteil
- 37: Klemmbackenteil
- 38: Schraubenbolzen
- 39: Öffnung
- 40: Oberfläche
- 41: zylindrischer Ansatz
- 42: Stelleinrichtung, Teleskopgestänge, Verlängerungseinrichtung
- 43: Klemmvorrichtungen
- 44: Gewinde
- 45: Hülse
- 46: Gestänge
- 47: Gewinde
- 48: Kohlefaserstränge
- 49: Kappe
- 50: Zapfen
- 51: Sechskantbohrung
- 53: Lagerschale
- 54: Flansch
- 55: Flansch
- 56: Lagerschale
- 57: Ausschnitt
- 59: Gelenkbolzen
- 60: Lagerschale
- 61: Gelenkbolzen
- 62: Gelenkkugelteile
- 63: Innenoberfläche
- 64: Pfeil
- 65: Bohrung
- 66: Mittelachse
- 68: Mittelachse
- 69: Stränge von Gestänge 2
- 70: Drehlager
- 71: Sicherungsringe

- H₁: Höhe Klemmteil 15
- H₂: Höhe Feder 4
- H₃: Abstand zwischen Oberfläche des Gestänges 2 und Innenoberfläche der Feder 4,
0,5 - 6 mm

## Patentansprüche

1. Fixateur (1) mit einem eine Anlagefläche (3) aufweisenden Tragkörper (2), auf dem zwei Feststellvorrichtungen zur Aufnahme von jeweils mindestens einem Knochenschrauben-Aufnahmeteil (7) angeordnet sind, wobei jede Feststellvorrichtung eine Drehsicherung und ein gegen axiales Verstellen sicherndes Feststellelement (13) aufweist, **dadurch gekennzeichnet, daß** die beiden Feststellvorrichtungen auf der gesamten Länge des Tragkörpers (2) mittels der Anlagefläche (3) drehgesichert sind.

2. Fixateur nach Anspruch 1, **dadurch gekennzeichnet, daß** der Tragkörper (2) im Querschnitt oval-, dreieck- oder sechskantförmig ausgebildet ist.

3. Fixateur nach Anspruch 1, **dadurch gekennzeichnet, daß** die Feststellvorrichtung einen Ansatz (41) aufweist, der mit einer oder mehreren Feststellschrauben (12') versehen ist, wobei zwischen zwei Verbindungselementen bzw. Kugelgelenkbacken (11') eine Stelleinrichtung bzw. ein Teleskopgestänge (42) vorgesehen ist, über die die Kugelgelenkbacken (11') auf dem Tragkörper axial verschiebbar sind.

4. Fixateur nach Anspruch 1 oder 2 **dadurch gekennzeichnet, daß** der als Gestänge ausgebildete Tragkörper (2) und ein Kugelgelenk (6) als Drehsicherung mindestens eine Anlagefläche (3) wie eine Nut zur Aufnahme eines Zwischenglieds (4) wie eine Feder aufweisen.

5. Fixateur nach einem oder mehreren der vorhergehenden Ansprüche, **dadurch gekennzeichnet, daß** das Zwischenglied (4) an seinen beiden gegenüberliegenden Enden je ein Feststellelement (13) aufweist, das zwischen dem Kugelgelenk (6) und dem Gestänge (2) eine Klemmverbindung herstellt, wobei das Feststellelement (13) des Zwischenglieds (4) aus einem Ringteil gebildet ist, das auf das Gestänge (2) und das Zwischenglied (4) axial aufbringbar ist und wobei das Feststellelement (13) des Zwischenglieds (4) eine Bohrung zur Aufnahme einer Feststellschraube (12) aufweist, die ein am Zwischenglied (4) fest angeordnetes Klemmteil (15) gegen die Oberfläche des Gestänges (2) preßt und das Kugelgelenk (6) gegen axiales Verschieben und Drehen sichert.

6. Fixateur nach einem oder mehreren der vorhergehenden Ansprüche, **dadurch gekennzeichnet, daß** das Klemmteil (15) an den beiden gegenüberliegenden Enden des Zwischenglieds (4) fest angeordnet ist und das Klemmteil (15) eine ebene, gegen die Oberfläche des Gestänges (2) andrückbare, plane Klemmfläche (20) aufweist, wobei das Klemmteil (15) eine Vertiefung (14) zur Aufnahme der Feststellschraube (12) aufweist und das Klemmteil (15) und/oder ein weiteres Stellelement (16) in seiner Bohrung mit einem Gewinde (21) versehen ist, das auf ein Gewinde (22) des Gestänges (2) aufschraubbar ist.

7. Fixateur nach einem oder mehreren der vorhergehenden Ansprüche, **dadurch gekennzeichnet, daß** dem Stellelement (16) ein Meßteil zugeordnet ist, wobei das Stellelement (16) gegen das Kugelgelenk (6) bzw. das dem Kugelgelenk zugeordnete Feststellelement (13) anpreßbar ist und wobei das Kugelgelenk (6) eine ballige Außenoberfläche (23) aufweist.

8. Fixateur nach einem oder mehreren der vorhergehenden Ansprüche, **dadurch gekennzeichnet, daß** das Kugelgelenk (6) die ballige Außenoberfläche (23) aufweist, auf der ein die Knochenschrauben (8) aufnehmendes Verbindungselement (11) verstellbar gelagert ist, wobei das Kugelgelenk (6) die ballige Außenoberfläche (23) aufweist, auf der das die Knochenschrauben (8) aufnehmende Verbindungselement (11) allseitig verschwenkbar gelagert ist.

9. Fixateur nach einem oder mehreren der vorhergehenden Ansprüche, **dadurch gekennzeichnet, daß** das Verbindungselement (11, 11') ringförmig ausgebildet und mindestens auf der einen Seite geschlitzt bzw. geteilt ist, wobei das Verbindungselement (11) ringförmig ausgebildet ist und zwei mit Abstand zueinander angeordnete Klemmbacken (24) mit koaxial zueinander ausgerichteten Bohrungen (25) aufweist, von denen zumindest die eine Bohrung als Gewindebohrung zur Aufnahme einer Klemmschraube (26) ausgebildet ist.

10. Fixateur nach einem oder mehreren der vorhergehenden Ansprüche, **dadurch gekennzeichnet, daß** auf der den Klemmbacken (24) gegenüberliegenden Seite ein Tragteil (27) zur Aufnahme des Knochenschrauben-Aufnahmeteils (7) vorgesehen ist.

11. Fixateur nach einem oder mehreren der vorhergehenden Ansprüche, **dadurch gekennzeichnet, daß** das Knochenschrauben-Aufnahmeteil (7) auf dem Tragteil (27) drehfest gelagert ist, oder das Knochenschrauben-Aufnahmeteil (7) auf dem Tragteil (27) drehbar gelagert und in verschiedenen Positionen feststellbar ist, wobei jedes Knochenschrauben-Aufnahmeteil (7) eine oder mehrere Knochenschrauben (8) trägt, wobei das Klemmbackenteil (36 oder 37) bzw. das Knochenschrauben-Aufnahmeteil (7) eine Öffnung oder eine Bohrung (29) aufweist, in die ein Ansatz (28) des Knochenschrauben-Aufnahmeteils (7) bzw. des Verbindungselements (11) einsetzbar und sicherbar ist.

12. Fixateur nach einem oder mehreren der vorhergehenden Ansprüche, **dadurch gekennzeichnet, daß** der Ansatz (28) zylindrisch und als Drehlager ausgebildet ist, oder der zylindrische Ansatz (28) als Festlager ausgebildet ist.

13. Fixateur nach einem oder mehreren der vorhergehenden Ansprüche, **dadurch gekennzeichnet, daß** ein Teil des Zwischenglieds (4), der in der Nut (3) aufgenommen oder gegenüber der Anlagefläche vorgesehen ist, im eingebauten Zustand des Zwischenglieds (4) einen Abstand (H₃) zu einer Oberfläche der Nut (3) oder der Anlagefläche aufweist.

14. Fixateur nach einem oder mehreren der vorhergehenden Ansprüche, **dadurch gekennzeichnet, daß** die Klemmfläche (20) des Zwischenglieds (4) außerhalb der Bohrung (30) des Kugelgelenks (6) vorgesehen ist, wobei die Klemmfläche (20) kleiner ist als die übrige sich an die Klemmfläche (20) anschließende Oberfläche (40) des Zwischenglieds (4), das mit Abstand (H₃) zur Oberfläche der Nut (3) angeordnet ist, wenn die Feststellvorrichtung auf dem Tragkörper (2) aufsitzt, wobei das Gestänge (2) und/oder die Knochenschrauben (8) längenveränderlich ausgebildet sind und wobei zumindest eine Kugelgelenkbacke (11") aus zwei Halbschalen besteht, die auf der einen Seite über ein Gelenk und auf der anderen Seite über eine Klemmschraube miteinander verbunden sind.

15. Fixateur nach einem oder mehreren der vorhergehenden Ansprüche, **dadurch gekennzeichnet, daß** die das Kugelgelenk (6) aufweisende Feststellvorrichtung zwei oder mehrere Feststellschrauben (12') aufweist, wobei an das Kugelgelenk (6) ein Ansatz zur Aufnahme des Arretierteils bzw. der Feststellschraube (12) einteilig angeordnet ist und wobei beiderseits des Kugelgelenks (6) je ein Ansatz (41) zur Aufnahme mindestens je eines Arretierteils bzw. mindestens je einer Feststellschraube (12) einteilig angeordnet ist.

16. Fixateur nach einem oder mehreren der vorhergehenden Ansprüche, **dadurch gekennzeichnet, daß** das Teleskopgestänge (42) über an den Kugelgelenkbacken (11') vorgesehene Klemmvorrichtungen (43) festsetzbar ist, wobei das Teleskopgestänge (42) aus einer ein Gewinde (44) aufweisenden Hülse (45) und einem ebenfalls ein Gewinde (47) aufweisenden Gestänge (46) besteht.

17. Fixateur nach einem oder mehreren der vorhergehenden Ansprüche, **dadurch gekennzeichnet, daß** der Tragkörper (2) aus Kohlefaser gebildet ist, die aus einzelnen, auf einem Kern spiralförmig aufwickelbaren Kohlefasersträngen besteht, die nach dem Aufwickeln auf den Kern zu einer bleibenden Querschnittsform verpreßt werden.

18. Fixateur nach einem oder mehreren der vorhergehenden Ansprüche, **dadurch gekennzeichnet, daß** das Kugelgelenk (6) aus zwei mittig teilbaren Lagerschalenhälften (53) besteht, auf die die Kugelgelenkbacken (11) radial aufbringbar sind.

19. Fixateur nach einem oder mehreren der vorhergehenden Ansprüche, **dadurch gekennzeichnet, daß** die eine Lagerschalenhälfte (53) einen ersten gegen die Außenseite der Kugelgelenkbacke (11) anlegbaren Flansch (54) und die zweite Lagerschalenhälfte (53) einen zweiten nach der Montage der Lagerschale (53) in eine Anlageposition gegen die Außenseite der Kugelgelenkbacke (11) bringbaren Flansch (55) aufweist, wobei die Lagerschale (56) des Verbindungselements (11) einen Ausschnitt (57) in Größe der Breite des Kugelgelenks (6) aufweist, so daß durch Drehen des Kugelgelenks (6) um seine Achse um 90° das Kugelgelenk (6) in die Lagerschale (56) einsetzbar und durch nochmaliges Drehen um 90° sicherbar ist.

## Claims

1. A fixator (1) with a carrier (2) having a contact surface (3) on which two fastening devices are arranged to each receive at least one bone screw receiving part (7), whereby each fastening device has a means to prevent rotation and a fastening element (13) to secure against axial shifting, **characterized in that** the two fastening devices are secured against rotation over the entire length of the carrier (2) by means of the contact surface (3).

2. The fixator according to Claim 1, **characterized in that** the cross section of the carrier (2) is oval, triangular or hexagonal in shape.

3. The fixator according to Claim 1, **characterized in that** the fastening device has a neck (41) that is provided with one or more fastening screws (12'), whereby between two connection elements or ball-and-socket joint jaws (11'), there is an adjustment device or a telescopic rod (42) via which the ball-and-socket joint jaws (11') can be slid axially on the carrier.

4. The fixator according to Claim 1 or 2, **characterized in that** the carrier (2) configured as a rod and a ball-and-socket joint (6) to prevent rotation have at least one contact surface (3) like a groove to receive an intermediate element (4) like a tongue.

5. The fixator according to one or more of the preceding claims, **characterized in that** the intermediate element (4) has a fastening element (13) on each of its two opposite ends that creates a clamping connection between the ball-and-socket joint (6) and the rod (2), whereby the fastening element (13) of the intermediate element (4) is made of an annular part that can be axially affixed on the rod (2) and on the intermediate element (4), and whereby the fastening element (13) of the intermediate element (4) has a bore to receive a fastening screw (12) which presses a clamping part (15) that is firmly positioned on the intermediate element (4) against the surface of the rod (2) and which secures the ball-and-socket joint (6) against axial shifting and rotation.

6. The fixator according to one or more of the preceding claims, **characterized in that** the clamping part (15) is firmly positioned on the two opposite ends of the intermediate element (4) and the clamping part (15) has a smooth, flat clamping surface (20) that can be pressed against the surface of the rod (2), whereby the clamping part (15) has an indentation (14) to receive the fastening screw (12) and the clamping part (15) and/or another adjustment element (16) is provided with a thread (21) in its bore that can be screwed onto a thread (22) of the rod (2).

7. The fixator according to one or more of the preceding claims, **characterized in that** a measuring part is associated with the adjustment element (16), whereby the adjustment element (16) can be pressed against the ball-and-socket joint (6) or against the fastening element associated with the ball-and-socket joint and whereby the ball-and-socket joint (6) has a convex outer surface (23).

8. The fixator according to one or more of the preceding claims, **characterized in that** the ball-and-socket joint (6) has the convex outer surface (23) on which a connection element (11) which receives the bone screw (8) is supported so that it can be adjusted, whereby the ball-and-socket joint (6) has the convex outer surface (23) on which the connection element (11) which receives the bone screws (8) is supported so that it can be pivoted in all directions.

9. The fixator according to one or more of the preceding claims, **characterized in that** the connection element (11, 11') is annular and is slit or divided on at least one side, whereby the connection element (11) is annular and has two clamping jaws (24) which are arranged at a certain distance from each other with bores (25) arranged coaxially with respect to each other, of which at least one bore is designed as a threaded bore for receiving a clamp screw (26).

10. The fixator according to one or more of the preceding claims, **characterized in that** a carrier (27) for receiving the bone screw receiving part (7) is provided on the side opposite from the clamping jaws (24).

11. The fixator according to one or more of the preceding claims, **characterized in that** the bone screw receiving part (7) part is supported on the carrier (27) in such a way that it cannot rotate, or the bone screw receiving part (7) is supported on the carrier (27) so that it can rotate and be fastened in various positions, whereby each bone screw receiving part (7) has one or more bone screws (8), whereby the clamping jaw part (36 or 37) or the bone screw receiving part (7) has an opening or a bore (29) into which a neck (28) of the bone screw receiving part (7) or of the connection element (11) can be inserted and secured.

12. The fixator according to one or more of the preceding claims, **characterized in that** the neck (28) is cylindrical and is designed as a pivoting support or the cylindrical neck (28) is designed as a fixed support.

13. The fixator according to one or more of the preceding claims, **characterized in that** a part of the intermediate element (4) that is held in the groove (3) or is provided across from the contact surface is at a distance (H₃) from a surface of the groove (3) or of the contact surface when the fastening device is in the built-in position.

14. The fixator according to one or more of the preceding claims, **characterized in that** the clamping surface (20) of the intermediate element (4) is provided outside of the bore (30) of the ball-and-socket joint (6), whereby the clamping surface (20) is smaller than the other surface (40) of the intermediate element (4) that is located adjacent to the clamping surface (20), said intermediate element (4) being arranged at a distance (H₃) from the surface of the groove (3) when the fastening device is positioned on the carrier (2), whereby the rod (2) and/or the bone screws (8) are designed so as to be variable in length, and whereby at least at least one ball-and-socket joint jaw (11") consists of two half-shells which are connected to each other on one side via a joint and on the other side via a clamping screw.

15. The fixator according to one or more of the preceding claims, **characterized in that** the fastening device having the ball-and-socket joint (6) has two or more fastening screws (12'), whereby a neck for receiving the locking part or the fastening screw (12) is attached in one piece to the ball-and-socket joint (6), and whereby on each side of the ball-and-socket joint, there is a neck (41) designed as one piece for receiving at least one locking part or at least one fastening screw (12).

16. The fixator according to one or more of the preceding claims, **characterized in that** the telescopic rod (42) can be secured by means of clamping devices (43) provided on the ball-and-socket joint jaws (11'), whereby the telescopic rod (42) consists of a sleeve (45) having a thread (44) and a rod (46) likewise having a thread (47).

17. The fixator according to one or more of the preceding claims, **characterized in that** the carrier (2) is made of carbon fiber consisting of individual carbon fiber strands that can be wound in a spiral onto a core and that are compressed into a permanent cross section shape after being wound onto the core.

18. The fixator according to one or more of the preceding claims, **characterized in that** the ball-and-socket joint (6) consists of two bearing shell halves (53) which can be split in the middle and on which the ball-and-socket joint jaws (11) can be installed radially.

19. The fixator according to one or more of the preceding claims, **characterized in that** one bearing shell half (53) has a first flange (54) that can be laid against the outside of the ball-and-socket joint jaw (11) while the second bearing shell half (53) has a second flange (55) that can be placed in a contact position against the outside of the ball-and-socket joint jaw (11) after the assembly of the bearing shell (53), whereby the bearing shell (56) of the connection element (11) has a cutout (57) the size of the width of the ball-and-socket joint (6) so that, by rotating the ball-and-socket joint (6) around its axis by 90°, the ball-and-socket joint (6) can be inserted into the bearing shell (56) and secured by rotating it by another 90°.

## Revendications

1. Fixateur (1) avec un corps porteur (2) présentant une face d'appui (3) sur laquelle sont disposés deux dispositifs d'arrêt destinés à recevoir chacun au moins une pièce de réception (7) de vis pour ostéosynthèse, chacun desdits dispositifs d'arrêt étant doté d'une protection contre la torsion et d'un élément de fixation (13) protégeant contre le désajustement axial, **caractérisé en ce que** les deux dispositifs d'arrêt sont protégés contre la torsion sur toute la longueur du corps porteur (2) grâce à la face d'appui (3).

2. Fixateur selon la revendication 1, **caractérisé en ce que** le corps porteur (2) présente une section ovale, triangulaire ou hexagonale.

3. Fixateur selon la revendication 1, **caractérisé en ce que** le dispositif d'arrêt présente une embase (41) pourvue d'une ou de plusieurs vis d'arrêt (12'), un organe de réglage ou un système de tiges télescopique (42) étant prévu entre deux éléments de raccordement ou mâchoires de rotule (11') permettant le déplacement des mâchoires de rotule (11') sur le corps porteur dans le sens axial.

4. Fixateur selon l'une des revendications 1 ou 2, **caractérisé en ce que** le corps porteur (2) conformé comme système de tiges et une rotule (6) présentent comme protection contre la torsion au moins une face d'appui (3) telle qu'une rainure pour la réception d'un composant intermédiaire (4) tel qu'un ressort.

5. Fixateur selon l'une ou plusieurs des revendications précédentes, **caractérisé en ce que** les deux extrémités opposées du composant intermédiaire (4) sont dotées d'un élément d'arrêt (13) qui établit une jonction par serrage entre la rotule (6) et le système de tiges (2), l'élément d'arrêt (13) du composant intermédiaire (4) consistant en une pièce annulaire qui peut être appliquée axialement sur le système de tiges (2) et le composant intermédiaire (4) et l'élément d'arrêt (13) du composant intermédiaire (4) présentant un alésage pour la réception d'une vis d'arrêt (12) qui presse un élément de serrage (15) solidaire du composant intermédiaire (4) contre la surface du système de tiges (2) et protège de cette façon la rotule (6) contre le déplacement axial et la torsion.

6. Fixateur selon l'une ou plusieurs des revendications précédentes, **caractérisé en ce que** l'élément de serrage (15) est disposé de façon fixe sur les deux extrémités opposées du composant intermédiaire (4) et que l'élément de serrage (15) présente une face de serrage plane (20) pouvant être pressée contre la surface du système de tiges (2), ledit élément de serrage (15) étant pourvu d'un creux servant à la réception de la vis d'arrêt (12) et l'élément de serrage (15) et/ou un autre élément de réglage (16) étant pourvu d'un taraudage (21) dans son alésage pouvant être vissé sur un filetage (22) de la tige (2).

7. Fixateur selon l'une ou plusieurs des revendications précédentes, **caractérisé en ce qu**'une pièce de mesure est assignée à l'élément de réglage (16), l'élément de réglage (16) pouvant être pressé contre la rotule (6) ou l'élément d'arrêt (13) assigné à la rotule et la rotule (6) présentant une surface extérieure bombée (23).

8. Fixateur selon l'une ou plusieurs des revendications précédentes, **caractérisé en ce que** la rotule (6) présente la surface extérieure bombée (23) sur laquelle est logé de façon réglable un élément de raccord (11) recevant les vis pour ostéosynthèse (8), la rotule (6) présentant la surface extérieure bombée (23) sur laquelle est logé de façon orientable dans toutes les directions l'élément de raccord (11) recevant les vis pour ostéosynthèse (8).

9. Fixateur selon l'une ou plusieurs des revendications précédentes, **caractérisé en ce que** l'élément de raccord (11, 11') se présente sous forme d'anneau qui est fendu ou divisé au moins sur un côté, l'élément de raccord (11) se présentant sous forme d'anneau et étant doté de deux mâchoires de serrage (24) espacées l'une de l'autre et pourvues d'alésages (25) orientés coaxialement l'un par rapport à l'autre, au moins l'un desdits alésages étant taraudé pour recevoir une vis de serrage (26).

10. Fixateur selon l'une ou plusieurs des revendications précédentes, **caractérisé en ce qu**'une pièce de support (27) destiné à porter l'élément de réception des vis à os (7) est prévue sur le côté opposé aux mâchoires de serrage (24).

11. Fixateur selon l'une ou plusieurs des revendications précédentes, **caractérisé en ce que** l'élément de réception des vis pour ostéosynthèse (7) est logé sur la pièce de support (27) de façon à résister à la torsion ou que l'élément de réception des vis pour ostéosynthèse (7) est logé de façon orientable sur la pièce de support (27) et peut être bloqué dans différentes positions, chaque élément de réception des vis pour ostéosynthèse (7) portant une ou plusieurs vis pour ostéosynthèse (8), et les mâchoires de serrage (36 ou 37) ou l'élément de réception des vis pour ostéosynthèse (7) présentant une ouverture ou un alésage (29) dans lequel on peut introduire et arrêter un embout (28) de l'élément de réception des vis pour ostéosynthèse (7) ou de l'élément de raccord (11).

12. Fixateur selon l'une ou plusieurs des revendications précédentes, **caractérisé en ce que** l'embout (28) est en forme de cylindre et conformé comme coussinet de pivotement ou que l'embout cylindrique (28) est conformé comme palier fixe.

13. Fixateur selon l'une ou plusieurs des revendications précédentes, **caractérisé en ce qu**'une partie du composant intermédiaire (4) qui est logée dans la rainure (3) ou prévue en face de la face d'appui présente, lorsque le composant intermédiaire (4) est monté, un écart (H₃) par rapport à une surface de la rainure (3) ou à la face d'appui.

14. Fixateur selon l'une ou plusieurs des revendications précédentes, **caractérisé en ce que** la face de serrage (20) du composant intermédiaire (4) est prévue à l'extérieur de l'alésage (30) de la rotule (6), ladite face de serrage (20) étant plus petite que la surface restante (40) contiguë à la face de serrage (20) du composant intermédiaire (4) qui est disposé avec un écart (H₃) par rapport à la surface de la rainure (3) lorsque le dispositif d'arrêt repose sur le corps porteur (2), le système de tiges (2) et/ou les vis pour ostéosynthèse (8) étant de longueur variable et au moins une mâchoire de rotule (11") consistant en deux demi-coquilles reliées d'un côté par une articulation et de l'autre par une vis de serrage.

15. Fixateur selon l'une ou plusieurs des revendications précédentes, **caractérisé en ce que** le dispositif d'arrêt présentant la rotule (6) est doté de deux ou plusieurs vis d'arrêt (12'), un embout servant à recevoir la pièce d'arrêt ou la vis d'arrêt (23) étant disposé sur la rotule (6) en formant avec celle-ci une seule pièce et un embout (41) servant à recevoir au moins une pièce d'arrêt ou au moins une vis d'arrêt (12) étant disposé des deux côtés de la rotule (6) en formant une seule pièce.

16. Fixateur selon l'une ou plusieurs des revendications précédentes, **caractérisé en ce que** le système de tiges télescopique (42) peut être fixé via les dispositifs de serrage (43) prévus aux mâchoires de rotule (11'), le système de tiges télescopique (42) étant composé d'une douille (45) avec taraudage (44) et d'une tige (46) avec filetage (47).

17. Fixateur selon l'une ou plusieurs des revendications précédentes, **caractérisé en ce que** le corps porteur (2) est en fibre de carbone qui consiste en des faisceaux de fibres de carbone individuels pouvant être enroulés en spirale sur un noyau et qui, après l'enroulement sur le noyau, sont moulés par compression pour obtenir une section de forme permanente.

18. Fixateur selon l'une ou plusieurs des revendications précédentes, **caractérisé en ce que** la rotule (6) est composée de deux demi-coussinets (53) divisibles en leur milieu sur lesquels on peut appliquer radialement les mâchoires de rotule (11).

19. Fixateur selon l'une ou plusieurs des revendications précédentes, **caractérisé en ce que** l'un des demi-coussinets (53) présente une première bride (54) pouvant être appliquée contre la surface extérieure de la mâchoire de rotule (11) et que le deuxième demi-coussinet (53) présente une deuxième bride (55) qui, après le montage du coussinet (53) peut être amenée dans une position d'appui contre la surface extérieure de la mâchoire de rotule (11), le coussinet (56) de l'élément de raccord (11) présentant une découpe (57) ayant la dimension et la largeur de la rotule (6), de sorte que, en tournant la rotule (6) de 90° autour de son axe, la rotule (6) puisse être insérée dans le coussinet (56) et, par une autre rotation de 90°, ladite rotule puisse être bloquée.
